# EUROPEAN PATENT APPLICATION

(11) **EP 1 783 217 A1**
(43) Date of publication of application: **09.05.2007**
(21) Application number: 05751236.0
(22) Date of filing: 17.06.2005
(51) Int. Cl.: C12N 15/09, C07K 14/395, C12C 11/00, C12G 1/02, C12N 1/19

(54) **STABILIZED PROLINE TRANSPORTER**

(30) Priority: 22.06.2004 JP 2004183220
(71) Applicant: SUNTORY LIMITED, Osaka-shi, Osaka 530-8203 (JP)
(72) Inventor: OMURA, Fumihiko, Suita-shi, Osaka 565-0824 (JP); FUKUI, Nobuyuki, Takatsuki-shi, Osaka 569-1123 (JP); KONDO, Hiroto, Kyoto-shi, Kyoto 606-0095 (JP)
(74) Representative: Vossius & Partner
(86) International application number: PCT/JP2005/011154
(87) International publication number: WO 2005/123917

(57) **Abstract**

The present invention relates to a stabilized proline transporter Put4 obtained by gene mutation and a gene encoding the same, as well as a *Saccharomyces* yeast strain which is obtained by yeast transformation with the gene and is capable of efficiently using proline in a source material such as wort. The stabilized mutated proline transporter Put4 can be used to achieve efficient use of nitrogen sources such as poorly assimilable proline contained in source materials (e.g., wort) for alcohol beverages. Fermentation using yeast capable of taking up a wide variety and large amounts of nitrogen sources facilitates carbon source assimilation and allows improvement of productivity for alcohol beverages. Moreover, the use of poorly assimilable nitrogen sources leads to resource savings and enables environmentally friendly production of alcohol beverages.

## Description

### TECHNICAL FIELD

The present invention relates to a stabilized proline transporter and a gene encoding the same, as well as to a *Saccharomyces* yeast strain which is obtained by yeast transformation with the gene and is capable of efficiently using proline in a source material such as wort.

### BACKGROUND ART

Proline is one of the major amino acids contained in source materials (e.g., wort) for alcohol beverages. However, in the presence of additional yeast's favorite nitrogen sources such as other amino acids or ammonia, proline is not actively taken up or assimilated by yeast cells. This relies on the fact that transcription of the proline transporter PUT4 gene (hereinafter referred to as "PUT4 gene"), wherein the proline transporter is responsible for proline uptake, is inhibited in the presence of, e.g., other amino acids or ammonia, and also relies on the very low stability of the proline transporter Put4 (hereinafter referred to as "Put4").

Under these circumstances, some attempts have been made to increase the use of proline etc. by yeast cells. Wine yeast is generally considered to not easily assimilate proline in grape juice because of its poor ability to transport proline into cells. For this reason, in the case of low nitrogen content in grape juice, it is necessary to add a nitrogen source such as diammonium phosphate for the sake of promoting fermentation, despite the presence of proline. JP 2001-346573 A reports that a single strain belonging to the genus *Saccharomyces,* which had the ability to assimilate proline, was obtained by attempting to screen a wide range of yeast groups for their ability to assimilate proline in grape juice. Moreover, this yeast strain has been used for wine brewing to achieve assimilation of proline in grape juice, which had not been possible for conventional yeast strains during their fermentation, thereby making it possible to produce a wine with a relatively high ethanol concentration, a rich deep taste and a mild aroma as the proline concentration is reduced.

On the other hand, analyses for amino acid sequence have also been attempted to study the stability of amino acid transporters. The tryptophan transporter Tat2 regulates tryptophan uptake through destabilization and degradation of the transporter itself. However, it is reported that the tryptophan transporter Tat2 is stabilized when 5 lysine resides in its N-terminal 31 amino acid residues are all replaced by arginines (Thomas Beck et al., The Journal of Cell Biology, Volume 146, Number 6, September 20, 1999 1227-1237).

In addition, Fumihiko Omura et al. (Biochemical and Biophysical Research Communications 287, 1045-1050 (2001)) report the results analyzed for the post-translational regulatory mechanism of transporter Bap2 playing an important role in the uptake of isoleucine, leucine and valine, indicating that the post-translational regulation requires lysine residues in the N-terminal 49 residues, etc.

Likewise, it is also known that the N-terminal lysine residues at positions 9 and 16 of the non-specific amino acid transporter Gap1p are involved in regulating its post-translational degradation. Gap1p is reported to be stabilized when these amino acids are replaced by arginine residues (Soetens, O. et al. J. Biol. Chem., vol. 276, 43949-43957 (2001)).

However, there is no report on amino acid residues involved in stabilization of Put4 responsible for uptake of proline which is one of the major amino acids contained in source materials (e.g., wort) for alcohol beverages. Thus, there is a need not only to improve the stability of Put4 for increased use of proline during fermentation, but also to create a brewing strain with high productivity which efficiently uses all amino acids, including proline, contained in source materials to be fermented (e.g., wort used as a source material for beer).
[Patent Document 1] JP 2001-346573 A
[Non-patent Document 1] The Journal of Cell Biology, Volume 146, Number 6, September 20, 1999 1227-123
[Non-patent Document 2] Biochemical and Biophysical Research Communications 287, 1045-1050 (2001)
[Non-patent Document 3] Soetens, O. et al. J. Biol. Chem., vol. 276, 43949-43957 (2001)

### DISCLOSURE OF THE INVENTION

### PROBLEMS TO BE SOLVED BY THE INVENTION

As stated above, although proline is one of the major amino acids contained in source materials (e.g., wort) for alcohol beverages, it is not actively taken up or assimilated by yeast cells in the presence of additional yeast's favorite nitrogen sources such as other amino acids or ammonia. This relies on the fact that transcription of the PUT4 gene related to proline uptake is inhibited in the presence of, e.g., other amino acids or ammonia, and also relies on the very low stability of Put4. The present invention aims to inhibit Put4 degradation by gene mutagenesis techniques (e.g., site-specific mutagenesis), thus ensuring an improved stability of the transporter, as well as to create a brewing strain with high productivity which efficiently uses all amino acids, including proline, contained in source materials to be fermented (e.g., wort used as a source material for beer).

### MEANS FOR SOLVING THE PROBLEMS

The present invention relates to a stabilized proline transporter obtained by gene mutation and a gene encoding the same, as well as a *Saccharomyces* yeast strain which is obtained by yeast transformation with the gene and is capable of efficiently using proline in a source material to be fermented (e.g., wort).

The inventors of the present invention have found that Put4 is stabilized when at least one of its N-terminal lysines at amino acid residues 9, 34, 35, 60, 68, 71, 93, 105 and 107 is replaced by arginine, preferably when at least six of these lysines are each replaced by arginine, and more preferably when all of these lysines are each replaced by arginine. The inventors have further found that yeast strains expressing this stabilized Put4 efficiently use all amino acids, including proline, contained in source materials to be fermented (e.g., wort used as a source material for beer) and also have high productivity. These findings led to the completion of the present invention.

Namely, the present invention is directed to a mutated proline transporter Put4 (hereinafter referred to as "mutant Put4"), in which at least one of the N-terminal lysines at amino acid residues 9, 34, 35, 60, 68, 71, 93, 105 and 107 is replaced by arginine.

The present invention is also directed to a mutant Put4, in which at least six of the N-terminal lysines at amino acid residues 9, 34, 35, 60, 68, 71, 93, 105 and 107 are each replaced by arginine.

The present invention is also directed to a mutant Put4, in which the N-terminal lysines at amino acid residues 60, 68, 71, 93, 105 and 107 are each replaced by arginine.

The present invention is also directed to a mutant Put4, in which all of the N-terminal lysines at amino acid residues 9, 34, 35, 60, 68, 71, 93, 105 and 107 are each replaced by arginine.

The present invention is also directed to a mutated proline transporter PUT4 gene (hereinafter referred to as "mutant PUT4 gene"), in which at least one of the genes encoding the N-terminal lysines at amino acid residues 9, 34, 35, 60, 68, 71, 93, 105 and 107 is replaced by a gene encoding arginine.

The present invention is also directed to a mutant PUT4 gene, in which at least six of the genes encoding the N-terminal lysines at amino acid residues 9, 34, 35, 60, 68, 71, 93, 105 and 107 are each replaced by a gene encoding arginine.

The present invention is also directed to a mutant PUT4 gene, in which the genes encoding the N-terminal lysines at amino acid residues 60, 68, 71, 93, 105 and 107 are each replaced by a gene encoding arginine.

The present invention is further directed to a mutant PUT4 gene, in which all of the genes encoding the N-terminal lysines at amino acid residues 9, 34, 35, 60, 68, 71, 93, 105 and 107 are each replaced by a gene encoding arginine.

The present invention is also directed to a recombinant vector containing the above gene or a yeast strain containing the recombinant vector, particularly a *Saccharomyces* yeast strain, and more particularly a beer yeast, wine yeast or sake yeast strain, etc.

The present invention is further directed to a method for producing beer, miscellaneous liquor or wine, which comprises performing a fermentation step in the presence of the above yeast strain.

### ADVANTAGES OF THE INVENTION

In the present invention, the stabilized mutant Put4 can be used to achieve efficient use of nitrogen sources such as non-assimilable proline contained in source materials (e.g., wort) for alcohol beverages. Fermentation using yeast capable of taking up a wide variety and large amounts of nitrogen sources facilitates carbon source assimilation and allows improvement of productivity for alcohol beverages. Moreover, the use of non-assimilable nitrogen sources leads to resource savings and enables environmentally friendly production of alcohol beverages.

### BRIEF DESCRIPTION OF DRAWINGS

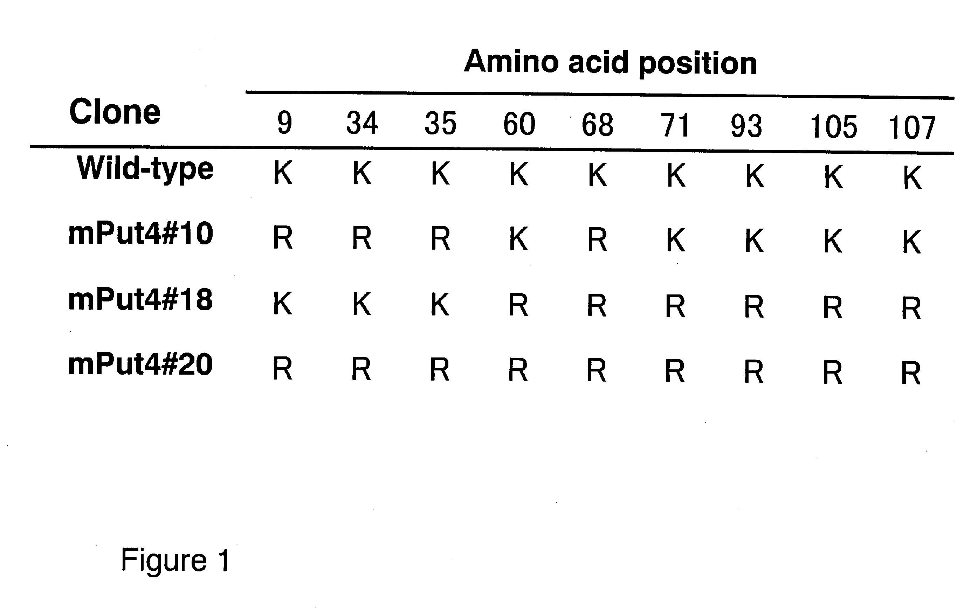

[Figure 1] Figure 1 schematically shows the sequences of three types of mutant Put4 obtained in Example 1. In mutant Put4, any or all of the lysine (K) residues at amino acid residues 9, 34, 35, 60, 68, 71, 93, 105 and 107 is/are replaced by an arginine (R) residue or residues.

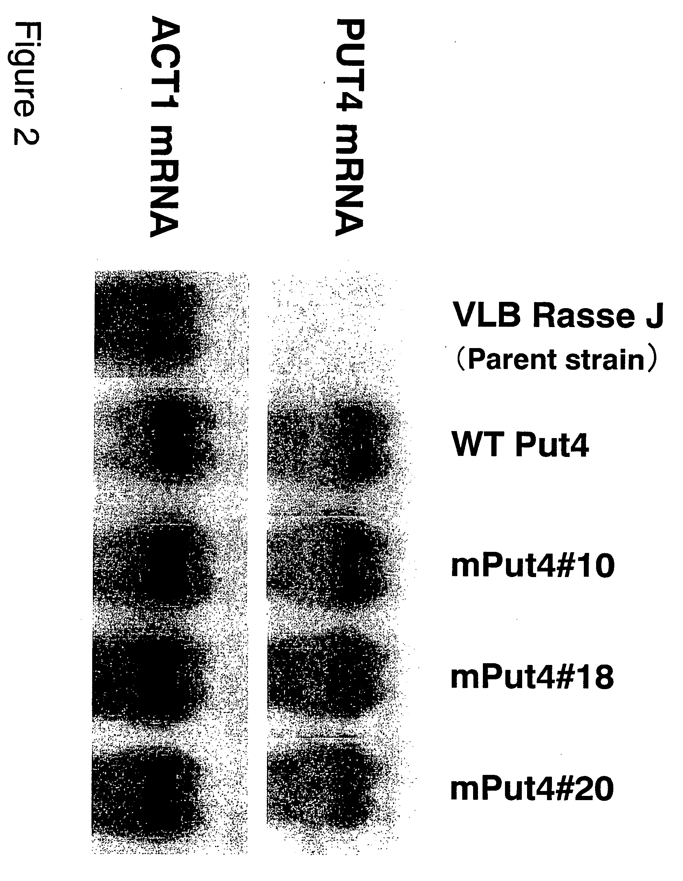

[Figure 2] Figure 2 shows PUT4 mRNA expressed in transformants of beer yeast bearing wild-type Put4 (WT Put4) and mutant Put4 obtained in Example 1 (mPut4#10, mPut4#18, mPut4#20), along with PUT4 mRNA expressed in their parent strain VLB Rasse J. ACT1 mRNA was also detected as an internal standard.

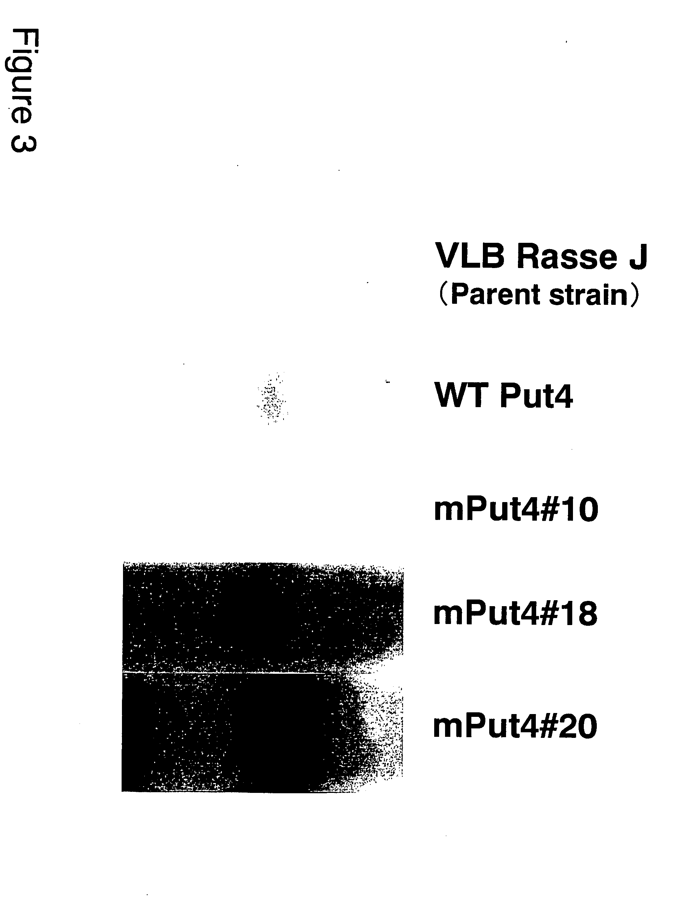

[Figure 3] Figure 3 shows the levels of Put4 expressed and accumulated in three transformants of beer yeast bearing the mutated Put4 proline transporters obtained in Example 1 (mPut4#10, mPut4#18, mPut4#20) and in a strain bearing wild-type Put4 (WT Put4). Each introduced Put4 is HA-tagged and can be detected by Western blotting with anti-HA antibody.

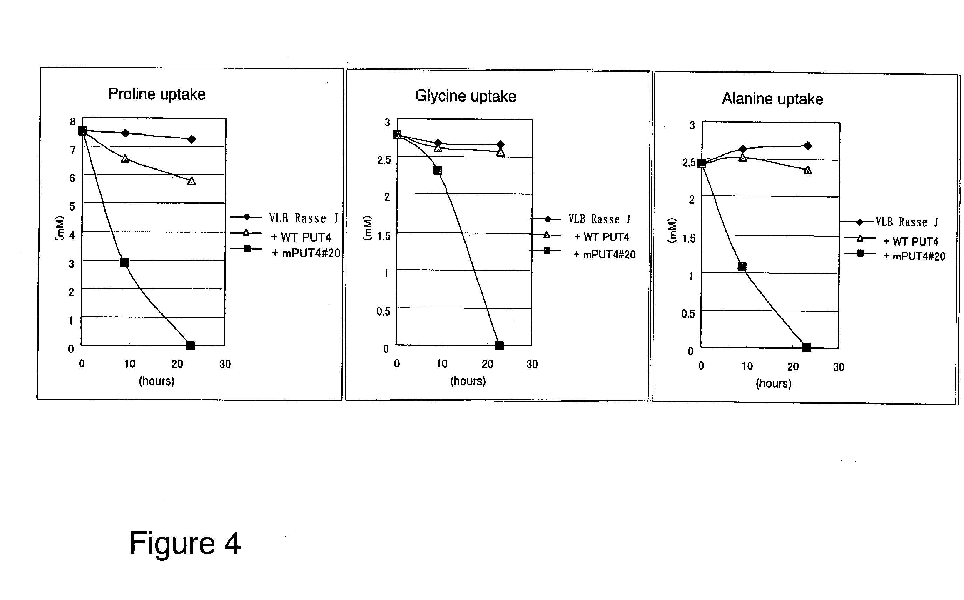

[Figure 4] Figure 4 shows the ability to take up and assimilate proline, glycine or alanine, which was measured for strains expressing mutant Put4 (mPUT4#20) and wild-type Put4 (WT PUT4) as well as their parent strain VLB Rasse J. These strains were aerobically cultured with shaking in a synthetic medium and measured for each amino acid level in the medium after 0, 9 and 23 hours. The results obtained were graphically plotted.

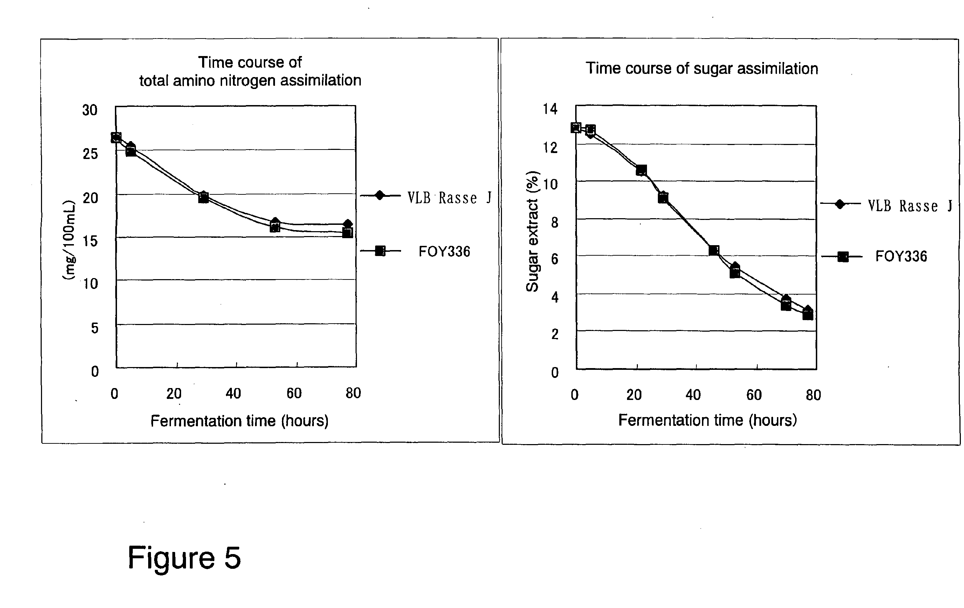

[Figure 5] Figure 5 graphically shows the time courses of sugar assimilation and total amino nitrogen assimilation during experimental fermentation for beer production using the strain expressing a mutated proline transporter mPut4#20 and its parent strain VLB Rasse J.

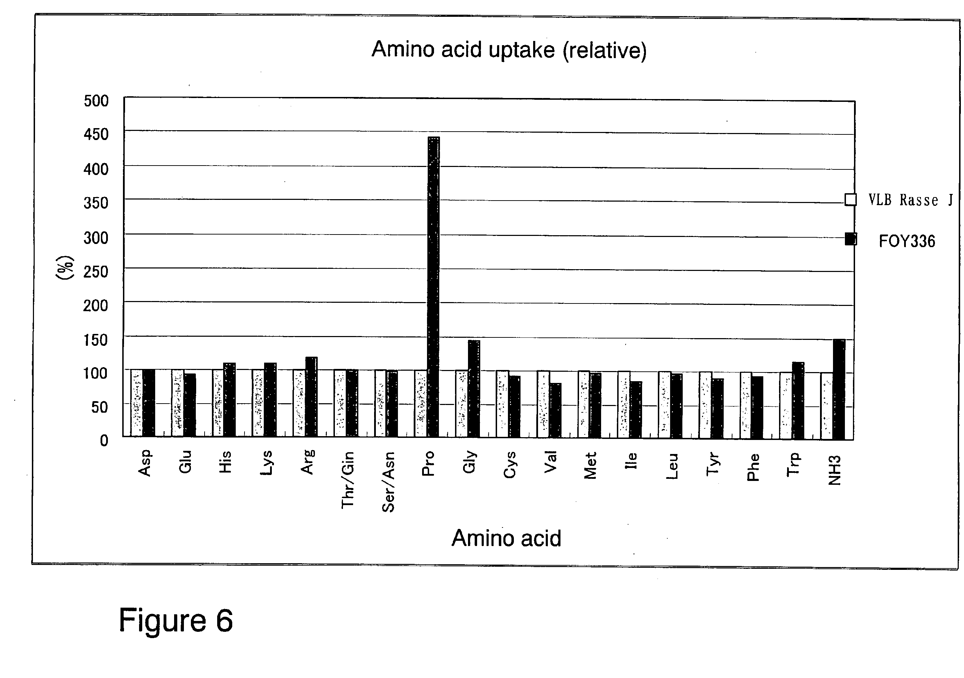

[Figure 6] Figure 6 shows the 77-hour uptake and assimilation of various amino acids during experimental fermentation for beer production using the strain expressing a mutated proline transporter mPut4#20 and its parent strain VLB Rasse J, assuming that the value of VLB Rasse J is set to 100%. Amino acids in the graph are expressed in three-letter abbreviations.

### BEST MODE FOR CARRYING OUT THE INVENTION

### <PUT4 gene>

The PUT4 gene of the yeast *Saccharomyces cerevisiae* has already been cloned and its nucleotide sequence has been reported (see, e.g., Gene, volume 83 (1989), 153-159, Vandenbol, M. et al.). Thus, the PUT4 gene can be obtained by PCR based on its nucleotide sequence information when chromosomal DNA prepared from the yeast *Saccharomyces cerevisiae* is used as a PCR template to amplify and isolate the PUT4 gene.

The chromosomal DNA used for PUT4 gene preparation may be prepared from any yeast strain as long as it belongs to *Saccharomyces cerevisiae* bearing the PUT4 gene. An example of a yeast strain belonging to *Saccharomyces cerevisiae* may be *Saccharomyces cerevisiae* X2180-1A (Rose, M.D., Winston, F. and Hieter, P. (1990): Methods in Yeast Genetics: A Laboratory Course Manual, Cold Spring Harbor Laboratory Press, Cold Spring Harbor, NY). PCR amplification with chromosomal DNA and the subsequent isolation of a target gene, including preparation of PCR primers, may be accomplished by any technique well known to those skilled in the art.

### <Site-specific mutagenesis>

Site-specific mutagenesis may be accomplished by any technique well known to those skilled in the art. As nonlimiting examples, the following may be used for site-specific mutagenesis: (1) Oligonucleotide-directed Dual Amber (ODA) method/Takara Biomedicals; (2) LA PCR in vitro mutagenesis/Takara Biomedicals; and (3) ExSite^{™} PCR-Based Site-Directed Mutagenesis Kit/STRATAGENE. A brief explanation will be given below for each technique.

### (1) Oligonucleotide-directed Dual Amber (ODA) method/Takara Biomedicals

A target gene is inserted into a plasmid bearing an amber mutation on the kanamycin resistance gene (Km) (e.g., pKF 18k-2/19k-2). The resulting DNA is converted into a single-stranded form by thermal denaturation, followed by simultaneous hybridization with a synthetic oligonucleotide for repairing the amber mutation on Km and with a synthetic oligonucleotide for mutagenesis by which a desired mutation is introduced into the target gene. This DNA is allowed to replicate while maintaining the introduced mutation, thus finally selecting only DNA in which the amber mutation on Km has been completely repaired. With high probability, the selected DNA has the desired mutation introduced into the target gene.

### (2) LA PCR in vitro mutagenesis/Takara Biomedicals

A DNA fragment to be mutated is inserted into a multicloning site of any plasmid. PCR (I) is performed using a primer for introducing a desired mutation into a target gene and a primer near the multicloning site. On the other hand, PCR (II) is performed over the whole of the inserted DNA fragment by using a primer for eliminating a single site (A) from the multicloning site in the direction opposite to mutagenesis in the target gene. The products from PCR (I) and (II) are mixed and subjected to PCR in such a way as to amplify the whole of the inserted DNA fragment bearing the introduced mutation. Among the DNA fragments thus obtained, those bearing the desired mutation lose the cloning site (A). Thus, when the PCR products are digested with a restriction enzyme (A) and then subcloned using the site (A), all the resulting recloned products theoretically have the desired mutation.

### (3) ExSite^{™} PCR-Based Site-Directed Mutagenesis Kit/STRATAGENE

A DNA fragment to be mutated is inserted into an appropriate plasmid. The resulting DNA is grown in dam+ E. coli cells (with DNA methylase activity), so that A in the GATC sequence is methylated. This plasmid is used as a template to synthesize, in both sense and antisense orientations, synthetic oligonucleotides for introducing a desired mutation. These oligonucleotides are used as primers for PCR. After PCR, the resulting DNA fragments are digested with a restriction enzyme DpnI (which digests only methylated DNA) to leave only a DNA fragment bearing the desired mutation. This fragment is ligated with T4 DNA ligase into the form of cyclic DNA, thus collecting a plasmid having the desired mutation introduced into a target gene.

In the present invention, for stabilization purposes, Put4 is mutated to replace a lysine residue(s) located near its N-terminal end by an arginine residue(s). Namely, the AAG or AAA codon encoding lysine is replaced by the AGG or AGA codon encoding arginine. The mutagenesis treatment may be confirmed by analyzing the nucleotide sequence of the mutated DNA using any technique well known to those skilled in the art.

In one embodiment of the present invention, a part of the open reading frame (ORF) obtained by PCR is cloned onto plasmid DNA and then subjected to mutagenesis by the Kunkel's method, so that one to all nine of the lysine residues located near the N-terminal end of Put4 (i.e., the lysine residues at amino acid residues 9, 34, 35, 60, 68, 71, 93, 105 and 107) is/are replaced by an arginine residue or residues. More specifically, synthetic DNA is prepared such that one to all nine of the lysine residues in Put4 located at amino acid residues 9, 34, 35, 60, 68, 71, 93, 105 and 107 is/are replaced by an arginine residue or residues. When mutagenesis is attempted to replace multiple lysine residues by arginine residues, a plurality of synthetic DNAs may be used as a mixture. Site-specific mutagenesis can be performed by the Kunkel's method (Kunkel, T.A., Proc. Natl. Acad. Sci. USA, 82, 488, 1985) using a Mutan-K kit (TaKaRa Shuzo Co., Ltd., Japan).

### <Yeast transformation>

Yeast transformation may be accomplished when the mutant PUT4 gene ORF as obtained above is cloned into a plasmid vector capable of constitutive expression in yeast and the resulting DNA construct is used to transform yeast cells. Although any type of yeast may be used, preferred are *Saccharomyces* yeast strains, particularly beer yeast, wine yeast and sake yeast strains.

In this process, any vector may be used to introduce the mutant PUT4 gene into yeast, as long as it is a plasmid vector capable of constitutive expression in yeast. For example, it is possible to use a chromosome integrative vector such as pUP3GLP (Omura, F. et al., FEMS Microbiol. Lett., 194, 207, 2001) or a single-copy replicating plasmid such as pYCGPY (Kodama, Y. et al., Appl. Environ. Microbiol., 67, 3455, 2001).

By integrating a drug resistance gene (e.g., a drug resistance gene against cycloheximide) into an expression plasmid, a transformant strain may be selected on agar medium containing the drug (e.g., 0.3 µg/ml).

Expression of the introduced mutant PUT4 gene in the resulting transformant strain can be examined by Northern blotting analysis of PUT4 mRNA expression levels. For example, a test strain is collected from its culture solution during the logarithmic growth phase. After washing, the yeast cells are suspended in 0.2 mM LETS buffer (0.1 M LiCl, 1%(w/v) SDS, 0.2 M Tris-HCl (pH 7.4), 0.01 M EDTA) and vigorously vortexed with 0.4 g glass beads to crush the cells. The resulting crushed cell suspension is centrifuged at 10,000 × g for 10 minutes to obtain the supernatant. The resulting supernatant is treated three times with phenol, followed by addition of ethanol to precipitate total RNA at -20°C. The resulting total RNA (20 µg) is developed by formaldehyde gel electrophoresis in a routine manner (Sambrook, J., Fritsch, E.F. and Maniatis, T. (1989): Molecular Cloning, Cold Spring Harbor Laboratory Press, Cold Spring Harbor, NY) and then transferred onto a nylon membrane to detect PUT4 mRNA. The detection will be performed using a DIG-Northern blotting detection kit (Roche).

The amount of mutant Put4 accumulated in the cells can be examined by Western blotting or other techniques. For example, a strain expressing mutant Put4 is collected from 10 ml culture solution during the logarithmic growth phase and crushed by vortexing with glass beads in lysis buffer (8 M urea, 5%(w/v) SDS, 40 mM Tris-HCl (pH 6.8), 0.1 mM EDTA, 1% β-mercaptoethanol) to obtain a cell extract. A sample of 60 µg total protein is developed by SDS-gel electrophoresis, transferred onto a nitrocellulose membrane and then Western blotted with rabbit polyclonal anti-HA antibody (Santa Cruz Biotechnology). When compared to wild-type Put4, mutant Put4 is prevented from being degraded and metabolized in the cells and is resistant to breakage. As a result, its amount accumulated in the cells is increased.

### <Mutant Put4>

Mutant Put4 of the present invention is a mutant in which at least one of the N-terminal lysines in Put4 located at amino acid residues 9, 34, 35, 60, 68, 71, 93, 105 and 107 is replaced by arginine. In another embodiment, mutant Put4 of the present invention is a mutant in which at least six of the above lysines are each replaced by arginine. In yet another embodiment, mutant Put4 of the present invention is a mutant in which all of the above lysines are each replaced by arginine.

Mutant Put4 of the present invention is characterized by high stability in yeast cells.

### <Culture using yeast cells constitutively expressing mutant Put4>

Amino acid uptake by yeast cells constitutively expressing mutant Put4 can be evaluated by performing aerobic culture under conditions suitable for the yeast cells and measuring the level of each amino acid in medium. The measurement of amino acids may be accomplished by any technique well known to those skilled in the art, for example by liquid chromatography. The yeast strain of the present invention shows improved proline uptake and also facilitates the uptake and assimilation of glycine and alanine.

When mutant Put4 is transformed into beer yeast, wine yeast or sake yeast strains, these yeast strains can be used to produce beer, miscellaneous liquor (including sparkling liquor), wine or Japanese rice wine (sake). Production of beer, miscellaneous liquor, wine or Japanese rice wine (sake) may be accomplished by any technique well known to those skilled in the art. Amino acid assimilation in each fermentation case may be evaluated by measuring the level of each amino acid in the fermentation solution.

### EXAMPLES

The present invention will be further described in more detail in the following examples, which are not intended to limit the scope of the invention.

### Example 1. Isolation and mutagenesis of PUT4 gene

The PUT4 gene of the yeast *Saccharomyces cerevisiae* has already been cloned and its nucleotide sequence has been reported. Thus, based on this information, the PUT4 gene was amplified by PCR and isolated. The PCR template used was chromosomal DNA prepared from the laboratory yeast X2180-1A (Rose, M.D., Winston, F. and Hieter, P. (1990): Methods in Yeast Genetics: A Laboratory Course Manual, Cold Spring Harbor Laboratory Press, Cold Spring Harbor, NY). In this case, for the purpose of examining the stability of a Put4 protein expressed later, PUT4 ORF was prepared such that 10 residues of the influenza virus hemagglutinin (HA)-derived amino acid sequence was added, as a tag, upstream of the ATG initiation codon in the PUT4 ORF (Chen, et al., Proc. Natl. Acad. Sci. USA, 90, 6508, 1993). As PCR primers for amplification of the PUT4 ORF, the following synthetic DNAs were used: 5'-GAGCTCATGT ACCCATACGA TGTTCCGGAT TACGCTAGCG TAAATATACT GCCCTTCCAC AAGA-3' (SEQ ID NO: 1) and 5'-GGATC CTTAC AACAA GGCGT CCAAG AACTT GTC-3' (SEQ ID NO: 2). The resulting PCR product of approximately 1.9 kb was cloned using a TOPO TA cloning kit (Invitrogen) to give plasmid pCR-PUT4.

For site-specific mutagenesis, a part of the PUT4 ORF was first prepared as a 408 bp SacI-KpnI fragment from pCR-PUT4 and inserted into the SacI/KpnI restriction enzyme site of pUC119 (TaKaRa Shuzo Co., Ltd., Japan) to give plasmid pUC-PUT4. Single-stranded DNA was prepared from pUC-PUT4 using helper phage or the like (Vieira, J. and Messing, J., Methods in Enzymology, 153, 3, 1987) and used as a template for site-specific mutagenesis. All of the following six synthetic DNAs were mixed together and used as a primer for mutagenesis such that some of the lysine residues in Put4p located at amino acid residues 9, 34, 35, 60, 68, 71, 93, 105 and 107 were replaced by arginines: 5'-GCCCT TCCAC AGGAA CAATA GACAC-3' (SEQ ID NO: 3); 5'-GGCGA CACCA GAAGG GAGGA GGATG T-3' (SEQ ID NO: 4); 5'-GCGAC AATGA AAGAG ATGAC GCCA-3' (SEQ ID NO: 5); 5'-TCCGT ATGGA GAGAA TATCT AGGAA CCAGT CCGC-3' (SEQ ID NO: 6); 5'-GGACT TGGAG AGATC GCCCT CC-3' (SEQ ID NO: 7); and 5'-GAGCC GCACA GACTA AGACA AGGTT TGCA-3' (SEQ ID NO: 8). It should be noted that the underlined regions in the above sequences of SEQ ID NOs: 3-8 each correspond to a replacement region that is introduced for mutation to a codon encoding arginine. Site-specific mutagenesis was performed by the Kunkel's method (Kunkel, T.A., Proc. Natl. Acad. Sci. USA, 82, 488, 1985) using a Mutan-K kit (TaKaRa Shuzo Co., Ltd., Japan). After the mutated DNAs were confirmed for their nucleotide sequences, it was indicated that genes encoding three mutated Put4 proline transporters (mPut4#10, mPut4#18, mPut4#20 (Figure 1)) were obtained. These genes were each collected in the form of double-stranded plasmid DNA and digested with SacI and KpnI to prepare a 408 bp SacI-KpnI fragment.

### Example 2. Expression of mutant PUT4 gene in beer yeast

The 408 bp SacI-KpnI fragments partially encoding the ORFs of the three mutant PUT4 genes were each inserted into the SacI/BglII site of an expression vector pUP3GLP together with a 1508 bp KpnI-BamHI fragment encoding the rest of the ORF, which had been prepared separately from pCR-PUT4, to create a plasmid for expression of mutant mPut4#10, #18 or #20 in beer yeast. As a control, the HA-tagged wild-type PUT4 ORF was prepared as a 1916 bp SacI-BamHI fragment and inserted into the SacI/BglII site of an expression vector pUP3GLP to create a plasmid for wild-type Put4 expression. The resulting expression plasmids were used to transform the beer yeast *Saccharomyces cerevisiae* strain VLB Rasse J by the lithium acetate method (Rose, M.D., Winston, F. and Hieter, P. (1990): Methods in Yeast Genetics: A Laboratory Course Manual, Cold Spring Harbor Laboratory Press, Cold Spring Harbor, NY). Since the expression plasmids used had a drug resistance gene against cycloheximide, transformant strains were selected on agar medium containing 0.3 µg/ml cycloheximide.

For the purpose of examining whether the resulting transformant strains expressed the introduced PUT4 gene, their expression levels of PUT4 mRNA were analyzed by Northern blotting. Each transformant strain expressing wild-type Put4 or mutant mPut4#10, mPut4#18 or mPut4#20 and the parent strain VLB Rasse J were each collected from 10 ml culture solution during the logarithmic growth phase. After washing, the yeast cells were suspended in 0.2 mM LETS buffer (0.1 M LiCl, 1%(w/v) SDS, 0.2 M Tris-HCl (pH 7.4), 0.01 M EDTA) and vigorously vortexed with 0.4 g glass beads and 0.15 ml phenol to crush the cells. The resulting crushed cell suspension was centrifuged at 10,000 × g for 10 minutes to obtain the supernatant. The resulting supernatant was treated three times with phenol, followed by addition of ethanol to precipitate total RNA at -20°C. The resulting total RNA (20 µg) was developed by formaldehyde gel electrophoresis in a routine manner (Sambrook, J., Fritsch, E.F. and Maniatis, T. (1989): Molecular Cloning, Cold Spring Harbor Laboratory Press, Cold Spring Harbor, NY) and then transferred onto a nylon membrane to detect PUT4 mRNA. The detection was performed using a DIG-Northern blotting detection kit (Roche). All of the transformant strains were found to highly express mRNA of the introduced PUT4 at the same level (Figure 2).

### Example 3. Evaluation of amount of mutant Put4 accumulated in cells

The strains expressing mutated proline transporters mPut4#10, mPut4#18 and mPut4#20 and wild-type transporter Put4 (hereinafter referred to as "wild-type Put4") were each collected from 10 ml culture solution during the logarithmic growth phase and crushed by vortexing with glass beads in lysis buffer (8 M urea, 5%(w/v) SDS, 40 mM Tris-HCl (pH 6.8), 0.1 mM EDTA, 1% β-mercaptoethanol) to obtain a cell extract. A sample of 60 µg total protein was developed by SDS-gel electrophoresis, transferred onto a nitrocellulose membrane and then Western blotted with rabbit polyclonal anti-HA antibody (Santa Cruz Biotechnology). Detection of HA-tagged wild-type Put4 and mutant Put4 was performed using chemiluminescence with an ECL kit (Amersham Biosciences). When compared to wild-type Put4, the mutated proline transporter mPut4#20 was prevented from being degraded and metabolized in the cells and was resistant to breakage. As a result, its amount accumulated in the cells was increased (Figure 3). Another mutated proline transporter mPut4#18 was also stabilized and protein accumulation was observed, but the degree of its stabilization was smaller than that of mPut4#20 (Figure 3).

### Example 4. Evaluation of ability to take up amino acids by strain expressing mutant Put4

The strain expressing mutant (mPut4#20) or wild-type Put4 and its parent strain VLB Rasse J were each inoculated at a cell concentration giving an absorbance of OD 600 nm = 0.5 into synthetic medium 2xSC [standard synthetic medium SC (Rose, M.D., Winston, F. and Hieter, P. (1990): Methods in Yeast Genetics: A Laboratory Course Manual, Cold Spring Harbor Laboratory Press, Cold Spring Harbor, NY) which had been modified such that only the nitrogen source was increased two-fold in concentration and proline was enriched to a final concentration of 7.5 mM] and then aerobically cultured with shaking at 30°C for 23 hours. The amino acid content in the cultured samples was quantified with an amino acid analyzer (Hitachi L-8800) at the beginning and after 9 and 23 hours. Although high expression of wild-type Put4 resulted in a certain effect on the improvement of proline uptake, the strain highly expressing the mutated proline transporter mPut4#20 showed greatly improved proline uptake and also facilitated the uptake of glycine and alanine (Figure 4).

### Example 5. Experimental fermentation for beer production using strain expressing mutant Put4

The yeast strain constitutively expressing mutant mPut4#20 (FOY336) was used for experimental fermentation in a 2 liter cylindrical tank using a 100% malt wort. Yeast was charged at a cell density of 15 × 10⁶ cells/ml relative to 1.5 liters of the wort and fermented at a temperature of 18°C. At the beginning of fermentation, the concentration of sugar extract was 12.87%(w/v), pH was 4.93, and dissolved oxygen was 8.6 ppm. Changes in sugar extract concentration during 77-hour fermentation were measured using a density meter (Anton Paar DMA-48), while simultaneously analyzing changes in total amino acid level (total amino nitrogen) remaining in the fermented wort. The time courses of sugar assimilation and total amino nitrogen assimilation are graphically shown for the parent strain VLB Rasse J and the strain FOY336 expressing mutant mPut4#20 (Figure 5). The graph indicated that the strain expressing mutant mPut4#20 facilitated the uptake and assimilation of both carbon and nitrogen sources as compared to its parent strain used as a control. When the 77-hour uptake of each amino acid by the strain expressing mutant mPut4#20 was expressed relatively to the uptake by its parent strain, the FOY336 strain expressing mPut4#20 was found to facilitate proline uptake 4-fold or more as compared to its parent strain (Figure 6). The table below shows analytical data of the beer made by the FOY336 strain expressing mutant mPut4#20.

**[Table 1]**

| Strain used | VLB Rasse J | FOY336 |
|---|---|---|
| Proline transporter | - | mPut4#20 |
| Alcohol (w/w%) | 4.4 | 4.5 |
| Fermentation degree (%) | 80.7 | 81.9 |
| Total amino nitrogen (mg/100 ml) | 16.4 | 15.1 |
| Ethyl acetate(ppm) | 28.9 | 36.4 |
| Normal propanol (ppm) | 12.8 | 13.8 |
| Isobutanol (ppm) | 9.5 | 11.6 |
| Isoamyl acetate(ppm) | 2.1 | 2.6 |
| Amyl alcohol (ppm) | 52.5 | 58.0 |

The above results indicated that the beer made using the strain expressing mutant mPut4#20 had a high degree of alcohol fermentation and was rich in desiable aromas of higher alcohols and esters, etc. Moreover, when using strains into which other mutated transporters mPut4#10 and mPut4#18 were introduced, the resulting beers were also found to have a clear and preferable taste with less unwanted tastes.

## Claims

1. A mutated proline transporter Put4, in which at least one of the N-terminal lysines at amino acid residues 9, 34, 35, 60, 68, 71, 93, 105 and 107 is replaced by arginine.

2. The mutated proline transporter Put4 according to claim 1, in which at least six of the N-terminal lysines at amino acid residues 9, 34, 35, 60, 68, 71, 93, 105 and 107 are each replaced by arginine.

3. The mutated proline transporter Put4 according to claim 2, in which the N-terminal lysines at amino acid residues 60, 68, 71, 93, 105 and 107 are each replaced by arginine.

4. The mutated proline transporter Put4 according to claim 1, in which all of the N-terminal lysines at amino acid residues 9, 34, 35, 60, 68, 71, 93, 105 and 107 are each replaced by arginine.

5. A mutated proline transporter PUT4 gene, in which at least one of the genes encoding the N-terminal lysines at amino acid residues 9, 34, 35, 60, 68, 71, 93, 105 and 107 is replaced by a gene encoding arginine.

6. The gene according to claim 5, in which at least six of the genes encoding the N-terminal lysines at amino acid residues 9, 34, 35, 60, 68, 71, 93, 105 and 107 are each replaced by a gene encoding arginine.

7. The gene according to claim 6, in which the genes encoding the N-terminal lysines at amino acid residues 60, 68, 71, 93, 105 and 107 are each replaced by a gene encoding arginine.

8. The gene according to claim 5, in which all of the genes encoding the N-terminal lysines at amino acid residues 9, 34, 35, 60, 68, 71, 93, 105 and 107 are each replaced by a gene encoding arginine.

9. A recombinant vector containing the gene according to any one of claims 5, 6, 7 and 8.

10. A yeast strain containing the recombinant vector according to claim 9.

11. The yeast strain according to claim 10, which is a *Saccharomyces* yeast strain.

12. The yeast strain according to claim 11, wherein the *Saccharomyces* yeast strain is a beer yeast or wine yeast strain.

13. A method for producing beer or wine, which comprises the step of performing fermentation with use of the yeast strain according to claim 11.
